(19) Europäisches Patentamt
European Patent Office
Office européen des brevets

(11) **EP 2 768 447 B2**

(12) # NEUE EUROPÄISCHE PATENTSCHRIFT
Nach dem Einspruchsverfahren

(45) Veröffentlichungstag und Bekanntmachung des Hinweises auf die Entscheidung über den Einspruch:
**16.09.2020   Patentblatt 2020/38**

(45) Hinweis auf die Patenterteilung:
**21.03.2018   Patentblatt 2018/12**

(21) Anmeldenummer: **12781061.2**

(22) Anmeldetag: **22.10.2012**

(51) Int Cl.:
***A61F 9/008*** (2006.01)

(86) Internationale Anmeldenummer:
**PCT/EP2012/070896**

(87) Internationale Veröffentlichungsnummer:
**WO 2013/057318 (25.04.2013 Gazette 2013/17)**

(54) **ERZEUGUNG VON SCHNITTFLÄCHEN IN EINEM TRANSPARENTEN MATERIAL MITTELS OPTISCHER STRAHLUNG**

PRODUCING CUT SURFACES IN A TRANSPARENT MATERIAL BY MEANS OF OPTICAL RADIATION

PRODUCTION DE SURFACES DE COUPE DANS UN MATÉRIAU TRANSPARENT À L'AIDE D'UN RAYONNEMENT OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.10.2011   DE 102011085046**

(43) Veröffentlichungstag der Anmeldung:
**27.08.2014   Patentblatt 2014/35**

(60) Teilanmeldung:
**18156076.4 / 3 354 239**

(73) Patentinhaber: **Carl Zeiss Meditec AG**
**07745 Jena (DE)**

(72) Erfinder:
• **PAPASTATHOPOULOS, Evangelos**
**71638 Ludwigsburg (DE)**
• **STOBRAWA, Gregor**
**07743 Jena (DE)**
• **BISCHOFF, Mark**
**07749 Jena (DE)**

(74) Vertreter: **Patentanwälte Geyer, Fehners & Partner mbB**
**Perhamerstrasse 31**
**80687 München (DE)**

(56) Entgegenhaltungen:
| EP-A1- 1 486 185 | EP-A1- 1 486 185 |
| WO-A2-2006/074469 | WO-A2-2006/074469 |
| DE-A1-102009 012 873 | US-A1- 2007 010 804 |
| US-A1- 2007 106 285 | US-A1- 2011 022 036 |
| US-A1- 2011 022 036 | US-A1- 2011 071 509 |

EP 2 768 447 B2

## Beschreibung

Die Erfindung bezieht sich weiter auf eine Bearbeitungsvorrichtung zur Erzeugung einer Schnittfläche in einem transparenten Material, die umfasst eine Lasereinrichtung, die dazu ausgebildet ist, mittels optischer Strahlung eine Trennung innerhalb des transparenten Materials zu bewirken, und die aufweist eine Optik, die optische Strahlung längs einer optischen Achse in einen im Material gelegenen Fokus bündelt und im Material ein Bildfeld hat, in dem der Fokus liegt und das eine Bildfeldgröße hat, eine Einrichtung zur Verstellung einer Lage des Fokus quer zur optischen Achse und längs der optischen Achse, eine Steuereinrichtung, welche mit der Lasereinrichtung verbunden ist und die Lasereinrichtung so ansteuert, dass deren Einrichtung zur Verstellung der Lage des Fokus die Lage des Fokus der optischen Strahlung im Material entlang einer Bahnkurve verschiebt, wobei die Steuereinrichtung die Lasereinrichtung so ansteuert, dass die Schnittfläche sich parallel zur optischen Achse erstreckt, in Projektion längs der optischen Achse eine Kurve ist und in Projektion längs der optischen Achse eine Maximalausdehnung aufweist. Diese Vorrichtung wird insbesondere im Gebiet der Ophthalmologie verwendet und ist z.B. aus der US 2011/0022036 A1 bekannt.

Bei diesen Anwendungsgebieten, wie auch bei anderen Applikationen, wirkt die optische Strahlung im Inneren des Materials, beispielsweise des Gewebes, das für die optische Strahlung transparent ist. Üblicherweise werden nicht-lineare Prozesse eingesetzt, welche eine Fokussierung von Bearbeitungsstrahlung, üblicherweise gepulste Laserstrahlung, in das Material hinein, d. h. unter die Oberfläche des Materials, benötigen. Die Erzeugung einer Schnittfläche geschieht dadurch, dass die Lage des Fokus im Material verschoben wird. In dem Verständnis, das dieser Beschreibung zu Grunde liegt, bedingt die Verschiebung des Fokus nicht zwingend, dass zu diesem Zeitpunkt auch Strahlung in den Fokus abgegeben wird. Insbesondere beim Einsatz gepulster Laserstrahlung wird der Fokus kontinuierlich verschoben und es werden nur zu gewissen Zeitpunkten während der Fokusverschiebung Laserstrahlungspulse abgegeben. Die entsprechenden Optiken bzw. Einrichtungen zu Fokusverschiebungen arbeiten dennoch kontinuierlich, weshalb unter dem Begriff "Fokusverschiebung" hier auch die entsprechende Verschiebung des Punktes verstanden wird, an den optische Strahlung fokussiert würde, auch wenn eine solche Strahlung momentan nicht abgegeben wird, z. B. zwischen zwei Laserpulsen.

Die hohe Fokussierung der Laserstrahlung, d. h. ein geometrisch stark begrenzter Fokus ist für nicht lineare Wirkungen von großer Bedeutung, da nur dann die erforderlichen Leistungsdichten im Material erreicht werden können. Dies gilt sowohl für nicht lineare Prozesse, bei denen ein einzelner Fokus bereits eine Wechselwirkung zu Folge hat, als auch für Prozesse, bei denen mehrere Laserstrahlungspulse, die hintereinander abgegeben werden, zusammenwirken, um einen Material trennenden Effekt zu erreichen. Es sind diesbezüglich auch Ansätze bekannt, bei denen an mehreren überlappenden Fokusstellen Laserstrahlungspulse abgegeben werden und erst im Überlappungsbereich die Zusammenwirkung der mehreren Laserstrahlungspulse zur Materialtrennung führt.

Dreidimensionale Schnittflächen, die sich parallel zur optischen Achse des Strahleinfalls (der sog. Hauteinfallsrichtung) erstrecken, sind z. B. als zylindermantelförmige Schnittflächen im Gebiet der Ophthalmologie, insbesondere bei der Katarakt-Operation, gefordert. Hier wird eine kreisrunde Öffnung in der Vorderseite des Kapselsacks mit einem bestimmten Durchmesser erzeugt. Die Schnittflächenform ist dann ein Kreiszylinder, welcher ungefähr parallel zur optischen Achse und somit auch parallel zur Haupteinfallsrichtung der optischen Strahlung orientiert ist.

Die EP 1486185 betrifft ein Gerät für die Katarakt-Operation, bei dem die Laserstrahlung mit Hilfe einer optischen Lichtleitfaser zum Handteil geleitet wird. Im Handteil wird ein Kollimator mechanisch verstellt, um die Lage des Fokus längs der optischen Achse zu verstellen. Die Fokusverstellung längs der optischen Achse kann deshalb nur sehr langsam erfolgen, da eine schnelle Bewegung des Kollimators unerwünschte Vibrationen und eine thermische Belastung des Handteiles zur Folge hätte. Darüber hinaus ist die Verwendung einer Lichtleitfaser bei den Leistungen, die zur Augenchirurgie nötig sind, äußerst problematisch.

In den US 7486409 und US 6590670 wird eine Methode zur schnellen Variation der Tiefenlage eines Fokus auf Basis einer schwingenden Stimmgabel beschrieben. Dabei wird mindestens eine Linse einer optischen Anordnung an den vibrierenden Arm einer Stimmgabel befestigt, welche durch einen Elektromagnet in Schwingungen versetzt wird. Die DE 10034251 schlägt zur Tiefenverstellung vor, einen Eckreflektor am schwingenden Arm einer Stimmgabel anzubringen. Der Eckreflektor wird mit einem nicht-kollimierten Lichtbündel beleuchtet, und durch Verstellung der Lage des Eckreflektors wird die Ausbreitung des rückreflektierten Lichtbündels variiert. Fokussiert man dieses Lichtbündel mit einem Objektiv, erhält man eine schnelle Variation der Fokuslage in Tiefenrichtung, d.h. längs der optischen Achse. Diese Stimmgabelanordnungen wurden für die optische Messtechnik vorgeschlagen.

Zum Erzeugen dreidimensionaler Schnittflächen, die sich parallel zur optischen Achse des Strahleinfalls erstrecken, sind Laserbearbeitungsvorrichtungen bekannt, welche eine Optik sowie eine Einrichtung zur dreidimensionalen Fokusverstellung aufweisen. Solche Bearbeitungsvorrichtungen verstellen den Fokus im Bildfeld und ergänzen diese zweidimensionale Verstellung durch eine Verstellung der Bildfeldebene zur dreidimensionalen Fokuslageneinstellung. Da die Verstellung der Bildfeldebene sehr viel langsamer ist, als die zweidimensionale Fokuslagenverstellung in der Bildebene, wird mit solchen Geräten darauf geachtet, dass die Bildebenenverstellung möglichst wenig benötigt wird, um die Schnittflächenerzeugung möglichst schnell zu gestalten. Es sind deshalb aus dem Stand der Technik beispielsweise spiralförmige Bahnkurven bekannt, welche eine schnelle Verstellung in der Bildebene mit einer vergleichsweise langsamen Verschiebung oder Verstellung der Bildebenenlage kombinieren. Auf diese Weise können beispielsweise zylinderförmige Schnittflächen, die parallel zur optischen Achse liegen, sehr schnell erzeugt werden.

Nachteilig an diesem Ansatz ist es, dass die Optik so gestaltet werden muss, dass innerhalb des Bildfeldes eine schnelle zweidimensionale Fokuslagenverstellung möglich ist. Auch muss die Bildfeldgröße so gestaltet werden, dass gewünschte Schnittflächengrößen abgedeckt werden.

Der Erfindung liegt deshalb die Aufgabe zu Grunde, die Bearbeitungsvorrichtung der eingangs genannten Art so weiterzubilden, dass aufwandsgering Schnittflächen erzeugt werden können, die im Wesentlichen parallel zur optischen Achse verlaufen, insbesondere zylindermantelförmige Schnittflächen.

Die Erfindung ist im Anspruch 1 definiert.

Eine Bearbeitungsvorrichtung zur Erzeugung einer Schnittfläche in einem transparenten Material, umfasst eine Lasereinrichtung, die dazu ausgebildet ist, mittels optischer Strahlung eine Trennung innerhalb des transparenten Materials zu bewirken, und die aufweist eine Optik, die optische Strahlung längs einer optischen Achse in einen im Material gelegenen Fokus bündelt und im Material ein Bildfeld hat, in dem der Fokus liegt und das eine Bildfeldgröße hat, eine Einrichtung zur Verstellung einer Lage des Fokus quer zur optischen Achse und längs der optischen Achse, eine Steuereinrichtung, welche mit der Lasereinrichtung verbunden ist und die Lasereinrichtung so ansteuert, dass deren Einrichtung zur Verstellung der Lage des Fokus die Lage des Fokus der optischen Strahlung im Material entlang einer Bahnkurve verschiebt, wobei die Steuereinrichtung die Lasereinrichtung so ansteuert, dass die Schnittfläche sich parallel zur optischen Achse erstreckt und in Projektion längs der optischen Achse eine Kurve ist, die eine Maximalausdehnung aufweist, wobei die Einrichtung zur Verstellung der Lage des Fokus quer zur optischen Achse eine Verschiebung des Bildfeldes quer zur optischen Achse bewirkt und die Steuereinrichtung die Lasereinrichtung so ansteuert, dass die Schnittfläche quer zur optischen Achse eine Maximalausdehnung hat, die größer ist als die Bildfeldgröße, und für die Einrichtung zur Verstellung der Lage des Fokus quer zur optischen Achse eine Bewegung der Lage des Fokus auf der Kurve vorgibt und längs der optischen Achse während der Bewegung entlang der Kurve eine mehrfach oszillierende Verstellung die Lage des Fokus zwischen einer oberen axialen Fokusposition und einer unteren axialen Fokusposition vorgibt.

Die Erfindung setzt eine Bahnkurve zur Schnittflächenerzeugung ein, welche vom üblichen Ansatz, der eine axiale Verstellung des Fokus möglichst zu minimieren sucht, grundlegend abweicht. Vielmehr wird nun die Lage des Fokus quer zur optischen Achse (sog. laterale Verstellung) sehr viel langsamer verstellt als axial, indem die Fokuslage auf der Kurve geführt wird, welche die Schnittfläche in Projektion längs der optischen Achse hat. Diese Kurve ist die Aufrisslinie der Schnittfläche. Es handelt sich bevorzugt um eine geschlossene Kurve, z. B. eine periodische Lissajous-Figur, beispielsweise einen Kreis, eine Ellipse etc. Senkrecht dazu, d. h. axial bzw. längs der optischen Achse wird der Fokus oszillierend verstellt. Man erhält somit eine Bahnkurve, welche in einer senkrecht zur optischen Achse gesehenen Draufsicht auf die Schnittfläche sich mäanderförmig zwischen dem unteren und dem oberen Rand der Schnittfläche entsprechend der unteren und der oberen axialen Fokusposition hin und her bewegt.

Die Schnittfläche, welche im Verfahren erzeugt wird, für welche die Steuerdaten ausgebildet sind und welche das Steuergerät der Bearbeitungsvorrichtung einstellt, erstreckt sich, wie bereits erwähnt, nicht zwingend strikt, sondern ggf. nur im Wesentlichen parallel zur optischen Achse. Abweichungen von der Parallelität die klein gegen die Länge der Kurve sind, sind zulässig. Insbesondere bei Lasereinrichtungen, die ein gegenüber der Ausdehnung des zu bearbeitenden Gebietes kleines Bildfeld haben und mit einer Bildfeldverstellung arbeiten, ist es möglich, innerhalb dieses kleinen Bildfeldes eine zusätzlich Fokusverstellung auszuführen. Diese erlaubt es natürlich, während das Bildfeld entlang der Kurve bewegt wird, die Lage des Fokus zusätzlich lateral auszulenken. Die Schnittfläche kann dadurch Bereiche haben. in denen keine strikte Parallelität zur optischen Achse gegeben ist. Gleichermaßen ist es möglich, eine zumindest abschnittsweise gegenüber der optischen Achse geneigte Schnittfläche zu erzeugen, indem die zusätzliche Fokusverstellung innerhalb des Bildfeldes so angesteuert wird, daß insgesamt die Schnittfläche leicht schräg liegt. In diesem Fall wird die zusätzliche laterale Fokusverstellung innerhalb des Bildfeldes geeignet zur oszillierenden axialen Fokusverstellung synchronisiert.

Die Bewegung des Fokus erfordert nicht zwingend, dass auf jede Fokusposition optische Strahlung zur Materialtrennung abgegeben wird. So ist es beispielsweise bei der Verwendung gepulster Strahlung sehr viel einfacher, den Fokus kontinuierlich zu verschieben, so dass die Spots auf die Laserstrahlungspulse abgegeben werden, im Material entsprechend der Bahngeschwindigkeit des Fokus beabstandet sind. Setzt man als materialtrennenden Effekt einen optischen Durchbruch ein, welcher eine Plasmablase erzeugt, ist es sogar bevorzugt, einen gewissen Abstand zwischen den Spots, auf die jeweils ein Laserstrahlungspuls abgegeben wird, einzuhalten. Es sind aber auch Verfahren bekannt, bei denen mehrere Laserstrahlungspulse zur Materialtrennung zusammenwirken, ohne einen optischen Durchbruch zu erzeugen. Diese Ansätze werden auch als Sub-Threshold-Verfahren bezeichnet.

Weiter schließt die Verstellung des Fokus gemäß der geschilderten Bahnkurve nicht aus, dass in einzelnen Abschnitten der Bahn, auf welcher der Fokus geführt wird, keine Laserstrahlung abgegeben wird, welche einen Material trennenden Effekt hat. Hier sind insbesondere bei der Verwendung einer plasmablasenbasierten Materialtrennung Abschnitte der Bahnkurve relevant, auf denen der Fokus von oben nach unten, d. h. in der Einfallsrichtung der optischen Strahlung bewegt wird. Erzeugt man Plasmablasen zur Materialtrennung, können in Gebieten, unterhalb der sich bildenden Plasmablase (die durchaus größer sein kann als der Fokus) zumindest für eine Zeitdauer keine weiteren optischen Durchbrüche und Plasmablasen erzeugt werden, da jede darüber liegende Plasmablase die Fokusqualität derart schwächt, dass ein optischer Durchbruch nicht mehr gesichert erreicht werden kann. Es ist deshalb im Rahmen der Erfindung durchaus möglich, in denjenigen Abschnitten der Kurve, in denen der Fokus nach unten, d. h. von der Lasereinrichtung wegbewegt wird, die optische Strahlung dunkelzutasten, d. h. abzuschalten oder zumindest hinsichtlich ihres Material bearbeitenden Effektes zu deaktivieren. Für solche Deaktivierungen sind im Stand der Technik verschiedene Ansätze bekannt, beispielsweise eine Laserpulsverlängerung, eine Fokusverschlechterung, spektrale Änderungen, Änderungen der Polarisation etc. Es ist deshalb im Rahmen der Erfindung ein Verfahren vorteilhaft, dass auf Abschnitten der Bahnkurve, in denen sich die Lage des Fokus mit (d. h. in gleicher Richtung) einer Einfallsrichtung der optischen Strahlung bewegt, die optische Strahlung abgeschaltet oder so modifiziert wird, dass die optische Strahlung keine Materialtrennungswirkung im transparenten Material hat.

Um die Schnittfläche auch in solchen Fällen möglichst dicht mit Abschnitten der Bahnkurve, in denen die optische Strahlung Materialtrennungswirkung hat, zu belegen, ist es zu bevorzugen, die Oszillation asymmetrisch zu gestalten, so dass Abschnitte der Bahnkurve, in denen sich die Lage des Fokus mit der Einfallsrichtung der optischen Strahlung bewegt, steiler verlaufen, als Abschnitte der Bahnkurve, in denen sich die Lage des Fokus entgegen der Einfallsrichtung der optischen Strahlung bewegt.

Die Schnittfläche kann im mathematischen Sinne zweifach zusammenhängend sein. Die Kurve ist dann geschlossen. Die erzeugte Schnittfläche kann insbesondere zylindermantelförmig sein, was bei Anwendungen an der erwähnten Katarakt-Operation vorteilhaft ist. Gleichermaßen kann die Schnittfläche natürlich auch zur Sektionierung oder Veränderung von transparentem Material, beispielsweise Augegewebe eingesetzt werden. In Frage kommen unter anderem die Sektionierung der Augenlinse vor der Entfernung bei der Katarakt-Operation oder die gezielte Schwächung der Kornea mit dem Ziel, dadurch die Hornhautkrümmung fehlsichtigkeitskorrigierend zu modifizieren. Insbesondere aber nicht ausschließlich für diese Anwendungen sind geschlossene, d. h. periodische Lissajous-Figuren geeignet, die sich kreuzen. Solche Lissajous-Figuren können erreicht werden, indem eine zweiachsige Ablenkung gemäß harmonischen Funktionen ausgeführt wird, die auf ganzzahligen Vielfachen einer Grundfunktion beruhen, wobei die ganzzahligen Vielfachen für die zwei Ablenkachsen unterschiedlich sind.

Die Schnittfläche kann aber auch einfach zusammenhängend sein; die Kurve ist dann eine nicht geschlossene Linie.

Wird an den axial oberen bzw. unteren Fokuspositionen eine Materialtrennung ausgeführt, definieren diese Fokuspositionen automatisch den oberen bzw. unteren Rand der Schnittfläche.

Die Verschiebung des Bildfeldes erlaubt einen Verzicht auf aufwendige Optiken, deren Bildfeld so groß ist, dass es das gesamte Bearbeitungsgebiet abdeckt. Es sei allerdings darauf hingewiesen, dass es durchaus möglich ist, die Bearbeitungsvorrichtung bzw. die Verfahren so zu modifizieren, dass die Verstellung der Lage des Fokus quer zur optischen Achse ohne Bildfeldverschiebung auskommt, wenn die Bildfeldgröße größer ist als die Maximalgröße der beabsichtigten, angestrebten oder erzeugten Schnittfläche. Eine derartige Erweiterung liegt im Rahmen der Erfindung. Man hat dann immer noch den Vorteil, dass die Fokusverstellung quer zur optischen Achse sehr viel langsamer ausgeführt werden kann, als längs der optischen Achse. Für die Anwendung in der Ophthalmologie sollte das Bildfeld im Auge dann einen Durchmesser von ca. 5 mm mindestens erreichen.

Verwendet man kleinere Bildfelder und möchte dennoch Strukturen erzeugen, die eine Maximalausdehnung quer zur optischen Achse haben, welche größer ist als die Bildfeldgröße, kann die Verschiebung des Bildfeldes besonders einfach durch eine Bewegung der Optik oder eines optischen Elementes quer zur optischen Achse bewirkt werden. Dies kann in Kombination mit einer lateralen Fokusverstellung innerhalb des Bildfeldes erfolgen.

Die Vorrichtung zur Erzeugung einer Schnittfläche ist besonders bei der Katarakt-Operation vorteilhaft anwendbar. Für solche Operationen benötigt man ein bewegliches Handteil, das am Auge aufgesetzt wird. Es ist deshalb bevorzugt, dass die Lasereinrichtung ein Basisteil und ein bewegliches Handteil aufweist, die miteinander über eine flexible oder gelenkige Übertragungseinrichtung verbunden sind, wobei die Einrichtung zur Verstellung der Lage des Fokus zweiteilig ausgebildet ist und einen ersten Scanner, der die Lage des Fokus längs der optischen Achse verstellt und im Basisteil angeordnet ist, und einen zweiten Scanner aufweist, der die Lage des Fokus quer zur optischen Achse verstellt und von dem zumindest eine Komponente im Handteil angeordnet ist. Für den zweiten Scanner zur Verstellung der Lage des Fokus quer zur optischen Achse ist diese Komponente bevorzugt ein Stellglied zur Verschiebung eines Objektivs quer zur optischen Achse.

Die Übertragungseinrichtung kann bevorzugt als Freistrahloptik ausgebildet sein. Es hat sich als vorteilhaft herausgestellt, wenn das Basisteil in die Übertragungseinrichtung ein nicht-kollimiertes Strahlenbündel einkoppelt, also ein Strahlenbündel, das einen bestimmten Divergenz- bzw. Konvergenzzustand hat. Die Übertragungseinrichtung, beispielsweise ein entsprechender Gelenkarm, führt dieses Strahlenbündel unter Beibehaltung des Divergenz- bzw. Konvergenzzustandes vom Basisteil zum Handteil. Die Querverschiebung des Objektives zur Realisierung des xy-Scanvorgangs ist dann kombiniert mit einer entsprechenden Aktivierung einer Divergenzverstelleinrichtung, welche die Divergenz oder Konvergenz des nicht-kollimierten Bündels beeinflusst, um eine Weglängenänderung, welche sich durch die Querverschiebung des Objektivs ergibt, zu kompensieren. Damit ist verhindert, dass die Querverschiebung des Objektivs, d.h. die Bildfeldverstellung zum xy-Scannen nicht unerwünscht eine Verstellung der Fokuslage längs der optischen Achse mit sich bringt.

Die Divergenzverstelleinrichtung kann besonders bevorzugt im Basisteil angeordnet werden, da dann das Handteil kompakt bleibt. Sie kann als Teleskop mit einem diesen verstellenden Aktuator realisiert sein. Die Verwendung eines nicht-kollimierten Strahlengangs zwischen Basisteil und Handteil hat weiter den Vorteil, dass besonders einfach der z-Scanner realisiert werden kann, indem der erste Scanner die Weglänge des nicht-kollimierten Strahlengangs verstellt. Für diese Weglängenverstellung kann ein Retro-Reflektor, beispielsweise ein Eckspiegel verwendet werden. Erst der Einsatz des nicht-kollimierten Strahlengangs erlaubt eine einfache Verstellung der Tiefenlage des Fokus durch Weglängenveränderung des Strahlengangs zum Objektiv. Dafür ist es allerdings wesentlich, dass zwischen dem ersten Scanner und dem Objektiv kein Parallelstrahlengang besteht, da dieser die Wirkung eines solchen ersten Scanners zunichtemachen würde. Der Strahlengang vom z-Scanner bis zum Objektiv ist folglich nichttelezentrisch. Bevorzugt umfasst das Basisteil einen nicht-kollimierten, d.h. divergierenden oder konvergierenden Strahlengangabschnitt und der erste Scanner umfasst einen Eckspiegel, der in diesem Strahlengangabschnitt liegt. Zur Verstellung der Lage des Fokus längs der optischen Achse wird der Eckspiegel verschoben, um die Länge des nicht-kollimierten Strahlengangabschnitts zu verändern. Die Verschiebung des Eckspiegels kann besonders schnell durch einen Schwinger erfolgen, der den Eckspiegel in Vibration zur periodischen Veränderung der Länge des nicht-kollimierten Strahlengangabschnittes versetzt. Alternativ ist es möglich, mehrere Eckspiegel auf einer rotierenden Scheibe zu montieren, die nacheinander im nicht-kollimierten Strahlengangabschnitt durchlaufen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen. Auch betrifft die Beschreibung von Verfahrensmerkmalen zur Materialtrennung bzw. zur Erzeugung von Steuerdaten gleichermaßen eine entsprechende Ausgestaltung der Steuereinrichtung, welche die Bearbeitungseinrichtung steuert. Umgekehrt sind Merkmale, welche hinsichtlich der Bearbeitungsvorrichtung insbesondere deren Steuereinrichtung beschrieben sind, gleichermaßen relevant für das entsprechende Verfahren zur Materialbearbeitung bzw. zur Steuerdatenerzeugung.

Die Steuerdatenerzeugung kann separat, d. h. unabhängig von der Bearbeitungsvorrichtung ausgeführt werden. Sie setzt natürlich entsprechende Kenntnis über die Bearbeitungsvorrichtung voraus, für welche die Steuerdaten vorgesehen werden. Die Erfindung wird durch die Ansprüche definiert. Nachfolgend wird die Erfindung beispielsweise anhand der beigefügten Zeichnungen, die auch erfindungswesentliche Merkmale offenbaren, noch näher erläutert. Es zeigen:

Fig. 1    eine Schemadarstellung einer Behandlungsvorrichtung für ophthalmologische Eingriffe, insbesondere zur Fehlsichtigkeitskorrektur,

Fig. 2    eine Schemadarstellung hinsichtlich des Aufbaus des Behandlungsgerätes der Fig. 1,

Fig. 3    eine Prinzipdarstellung zur Einbringung gepulster Laserstrahlung in das Auge mit dem Behandlungsgerät der Fig. 1,

Fig. 4    eine schematische Darstellung für eine Schnittfläche durch einen Kapselsack eines Auges,

Fig. 5    eine Schemadarstellung der in Fig. 4 dargestellten Schnittfläche,

Fig. 6    eine Schemadarstellung ähnlich der Fig. 5 zur Veranschaulichung der Führung des Laserstrahls auf einer Bahnkurve,

Fig. 7    eine Schemadarstellung einer Schnittfläche, die anders als die Schnittfläche der Fig. 4 kein Gebiet umschließt,

Fig. 8    eine Schemadarstellung ähnlich der Fig. 4 zur Verdeutlichung einer möglichen Anwendung der Schnittfläche der Fig. 7,

Fig. 9    eine Schemadarstellung eines Behandlungsgeräts, das zur Katarakt-Operation ausgebildet ist,

Fig. 10   eine Schemadarstellung eines Handteils des Behandlungsgeräts der Fig. 9,

Fig. 11   eine Schemadarstellung eines z-Scanners des Behandlungsgerätes der Fig. 9,

Fig. 12   eine schematische Blockdarstellung für das Steuerkonzept des Behandlungsgerätes der Fig. 9, und

Fig. 13 und 14 Schemadarstellungen für alternative Ausgestaltungen des z-Scanners des Behandlungsgeräts der Fig. 9.

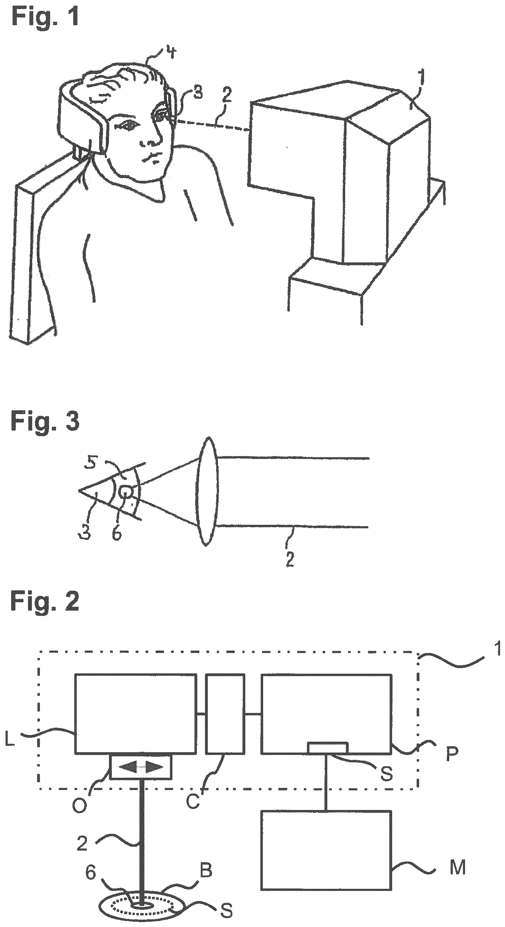

Fig. 1 zeigt ein Behandlungsgerät 1 für die Augenchirurgie. Damit kann z. B ein augenchirurgische Verfahren ausgeführt werden, das dem in EP 1 159 986 A2 bzw. US 5,549,632 beschriebenen ähnelt. Das Behandlungsgerät 1 erzeugt mittels Behandlungs-Laserstrahlung 2 eine Materialtrennung in transparentem Material. Diese Materialtrennung kann z. B. eine Schnittflächenerzeugung sein, insbesondere kann das Behandlungsgerät zur Fehlsichtigkeitskorrektur eine Veränderung an einem Auge 3 eines Patienten 4 bewirken. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Aberrationen höherer Ordnung umfassen. Die Materialtrennung kann auf dem Gebiet der Hornhautchirurgie aber auch an anderen Geweben des Auges eingesetzt sein, z. B. bei der Kataraktoperation. Soweit nachfolgend Bezug auf die Augenchirurgie genommen wird, ist das auf jeden Fall nur exemplarisch und nicht einschränken zu verstehen.

Die Baugruppen des Gerätes 1 werden im beschriebenen Ausführungsbeispiel von einer integrierten Steuereinheit gesteuert, die aber natürlich auch eigenständig ausgebildet sein kann.

Fig. 2 zeigt schematisch das Behandlungsgerät 1. Es weist in dieser Variante mindestens drei Einrichtungen oder Module auf. Eine Lasereinrichtung L gibt über eine Optik O den Laserstrahl 2 auf das Material, z. B. ein Auge ab und verstellt die Lage des Fokus im Material in drei Raumrichtungen. Die Verstellung längs der Haupteinfallsrichtung der optischen Strahlung (z-Achse) wird axiale Verstellung bezeichnet, die Verstellung senkrecht dazu als laterale Verstellung. In der in Fig. 2 gezeigten Variante hat die Optik O ein Bildfeld B, in dem ein Fokus 6 der Laserstrahlung 2 liegt, das kleiner ist als die Ausdehnung des zu bearbeitenden Gebietes. Zur lateralen Verstellung der Lage des Fokus 6 im Material wird das Objektiv O der Lasereinrichtung L quer zur optischen Achse verschoben wird. Dies ist in Fig. 2 durch einen Doppelpfeil für das Objektiv O angedeutet. Dadurch wird der Fokus 6 samt dem Bildfeld B verschoben. Optional kann der Fokus 6 zusätzlich in einem Bereich S innerhalb des Bildfeldes B feinverstellt werden.

Der Betrieb der Lasereinrichtung L erfolgt für alle Varianten vollautomatisch unter Steuerung durch eine integrierte oder separate Steuereinrichtung C. Die Lasereinrichtung L startet auf ein entsprechendes Startsignal hin die Ablenkung des Laserstrahls 2 und erzeugt dadurch Schnittflächen auf noch zu beschreibende Art und Weise.

Die Steuereinrichtung C arbeitet nach Steuerdaten, die entweder von ihr erzeugt wurden oder ihr zugeführt wurden. In letzterem Fall, der in Fig. 2 gezeigt ist, werden die für den Betrieb erforderlichen Steuerdaten der Steuereinrichtung C zuvor von einer Planungseinrichtung P als Steuerdatensatz über nicht näher bezeichnete Steuerleitungen zugeführt. Die Ermittlung oder Übertragung der Steuerdaten findet vor dem Betrieb der Lasereinrichtung L statt. Natürlich kann die Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

In der Augenheilkunde wird bevorzugt vor dem Einsatz des Behandlungsgerätes 1 die Fehlsichtigkeit des Auges 3 mit einer oder mehreren Messeinrichtungen M vermessen. Die Messwerte werden dann dem Steuereinrichtung bzw. der Planungseinrichtung P zugeführt und bilden die Basis für die Steuerdatenerzeugung. Insbesondere kann die Lage und/oder Ausdehnung eines zu bearbeitenden, insbesondere zu sektionierenden Bereichs gemessen werden.

Die Steuereinrichtung bzw. die Planungseinheit P erzeugt den Steuerdatensatz aus den Messdaten, die ermittelt wurden, z. B. für das zu behandelnde Auge. Sie werden der Planungseinheit P über eine Schnittstelle S zugeführt und stammen im dargestellten Ausführungsbeispiel aus einer Messeinrichtung M, die das Auge des Patienten 4 zuvor vermessen hat. Natürlich kann die Messeinrichtung M auf beliebige Art und Weise die entsprechenden Messdaten an die Planungseinrichtung P oder direkt an die Steuereinrichtung C übermitteln.

Die Laserstrahlung 2 wird in der beschriebenen Ausführungsform als gepulster Laserstrahl fokussiert in das Material, z. B. das Auge 3 gebracht. Die von der Lasereinrichtung L erzeugte Pulsdauer liegt dabei z. B. im Femtosekundenbereich, und die Laserstrahlung 2 wirkt mittels nicht-linearer optischer Effekte im Material, z. B. dem Kapselsack der, der Augenlinse oder der Hornhaut. Der Laserstrahl weist z. B. 50 bis 800 fs kurze Laserpulse (bevorzugt 100 - 400 fs) mit einer Pulswiederholfrequenz zwischen 10 kHz und 10 MHz auf. Die Art des Material trennenden Effektes, den das Behandlungsgerät 1 mit der Laserstrahlung 2 nutzt, ist jedoch für die nachfolgende Beschreibung nicht weiter relevant, insbesondere muss keine gepulste Laserstrahlung verwendet werden. Wesentlich ist lediglich, dass ein Fokus von Bearbeitungsstrahlung 2 im Material längs einer Bahnkurve verschoben wird. Alternativ kann UV-Strahlung (300 bis 400 nm), insbesondere mit einer Wellenlänge von ca. 355 nm und einer Pulsdauer zwischen 0,1 und 10 ns verwendet werden.

Das Behandlungsgerät 1 bildet im Material eine Schnittfläche aus, deren Form vom Muster abhängt, mit dem die Laserpulsfoki im Gewebe angeordnet sind/werden. Das Muster hängt wiederum von der Bahnkurve ab, entlang der der Fokus verschoben wird. Sie gibt Zielpunkte für die Fokuslage vor, an denen ein oder mehrere Laserpuls(e) abgegeben wird(werden), und definiert letztlich die Form und Lage der Schnittfläche.

Eine mögliche Wirkungsweise des Laserstrahls 2 ist in Fig. 3 schematisch angedeutet. Er wird mittels einer nicht näher bezeichneten Optik der Lasereinrichtung L in das Material, z. B. die Hornhaut 5 oder Linse des Auges fokussiert. Dadurch entsteht im Material ein Fokus 6, in dem die Laserstrahlungsenergiedichte so hoch ist, dass in Kombination mit der Pulslänge ein nichtlinearer Effekt auftritt. Beispielsweise kann jeder Puls der gepulsten Laserstrahlung 2 am jeweiligen Ort des Fokus 6 einen optischen Durchbruch im Material, z. B. in der Hornhaut 5 oder Linse erzeugen, welcher in Fig. 3 exemplarisch durch eine Plasmablase schematisch angedeutet ist. Dadurch wird aufgrund dieses Laserpulses Material, z. B. Gewebe getrennt. Bei Entstehung einer Plasmablase umfasst die Gewebeschichttrennung ein größeres Gebiet, als den Spot, welchen der Fokus 6 der Laserstrahlung 2 überdeckt, obwohl die Bedingungen zur Erzeugung des Durchbruches nur im Fokus erreicht werden. Damit von jedem Laserpuls ein optischer Durchbruch erzeugt wird, muss die Energiedichte, d. h. die Fluence der Laserstrahlung oberhalb eines gewissen wellenlängsabhängigen und pulslängenabhängigen Schwellwertes liegen. Dieser Zusammenhang ist dem Fachmann beispielsweise aus der DE 695 00 997 T2 bekannt.

Alternativ kann ein materialtrennender Effekt durch die gepulste Laserstrahlung auch dadurch erzeugt werden, dass mehrere Laserstrahlungspulse im einen Bereich abgegeben werden, wobei sich für mehrere Laserstrahlungspulse die Spots 6, d.h. Orte des Fokus 6 überlappen. Es wirken dann mehrere Laserstrahlungspulse zusammen, um einen gewebetrennenden Effekt zu erreichen, ohne dass Plasmablasen entstehen (sog. Sub-threshold-Verfahren). Beispielsweise kann das Behandlungsgerät 1 das Prinzip verwenden, das in der WO 2004/032810 A2 beschrieben ist.

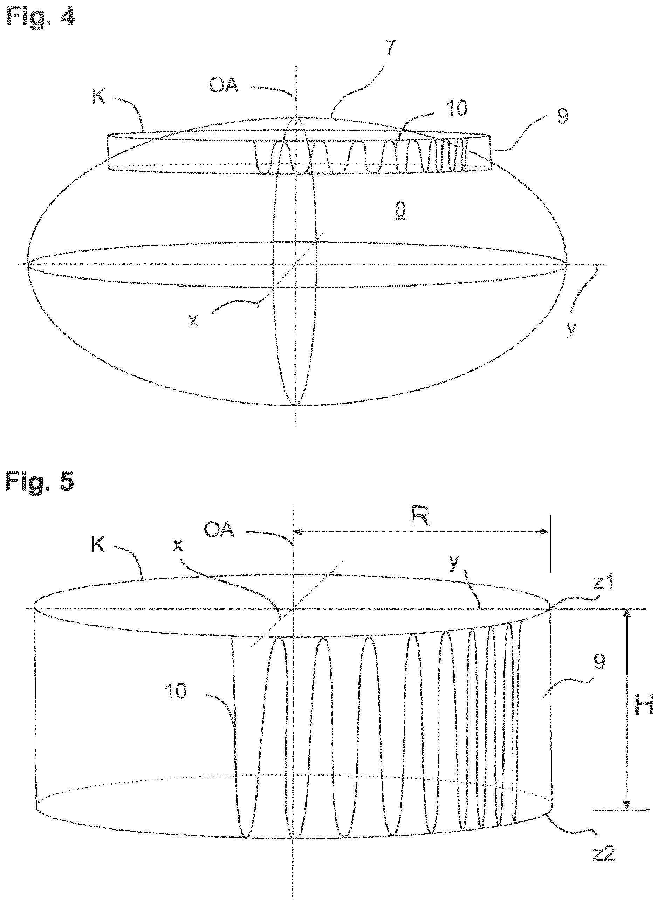

Fig. 4 zeigt exemplarisch die Erzeugung einer Schnittfläche 9 in einem Kapselsack, der eine Linse 8 des Auges 3 einhüllt. Die Schnittfläche 9 ist zweifach zusammenhängend. Sie hat die Form eines Kreiszylindermantels, also der Mantelfläche eines Zylinders, dessen Erzeugende eine kreisförmige Kurve K ist; jedoch sind auch andere geschlossene Figuren als Erzeugende des Zylinders möglich, insbesondere jede periodische Lissajous-Figur, auch sich kreuzende.

Die Kurve K liefert die Vorgabe für die laterale Verstellung des Fokus, also in der x/y-Ebene. Die entsprechenden Koordinaten x/y liegen in der Ebene des Bildfeldes B der Lasereinrichtung L. Sie sind in den Figuren exemplarisch eingetragen. Senkrecht zur optischen Achse OA, welche die Haupteinfallsrichtung der optischen Laserstrahlung 2 ist, - also axial - wird die Lage des Fokus 6 entlang der Kurve K verstellt. Währenddessen wird die axiale Lage des Fokus zugleich längs der optischen Achse OA, d. h. senkrecht zur x/y-Ebene, oszillierend verstellt. Dadurch ergibt sich eine Bahnkurve 10 in der Schnittfläche 9, die zwischen einer oberen axialen Fokusposition z1 und einer unteren axialen Fokusposition z2 hin und her oszilliert. Diese Oszillationen werden während der Bewegung entlang der Kurve K mehrfach ausgeführt.

Dieses Vorgehen vermeidet eine schnelle laterale Ablenkung der Laserstrahlung 2 über das zu bearbeitende Gebiet, welches im beschriebenen Fall durch den Radius R der Kurve K charakterisiert ist und eine laterale Maximalabmessung von 2*K hat. Optional wird eine Größe des Bildfelds B verwendet, die sehr viel kleiner ist als diese laterale Ausdehnung. Grundsätzlich wird eine schnelle axiale Bewegung ausgeführt, während die Verschiebung in der x/y-Ebene der Kurve K folgt. Somit werden im wesentlichen Schnitte erzeugt, die parallel zur optischen Achse liegen. Damit kann ein einfaches optisches System verwendet werden, das keiner schnellen lateralen Fokusverschiebung mit großer Amplitude bedarf.

Für die axiale Fokusverschiebung kommen mehrere Ansätze in Frage, beispielsweise eine elektrooptische Linse oder ein sogenannter zweistufiger z-Scanner, der eine langsame großhubige Verstellung mit einer schnellen kurzhubigen Verstellung kombiniert. Die beiden Stufen eines solchen zweistufigen z-Scanners können räumlich getrennt oder kombiniert ausgebildet sein.

Für den Fall der Bearbeitung der menschlichen Linse, die nachfolgend exemplarisch abgehandelt werden soll, ist ein maximaler Wert NA = 0,2 aus anatomischen Gründen nicht wesentlich überschreitbar. Daraus ergibt sich die effektive Brennweite des verwendeten Objektivs zu mindestens etwa

$$f \approx \frac{n \cdot D}{2 \cdot NA} \approx \frac{1.3 \cdot 6mm}{2 \cdot 0.2} \approx 19mm$$

Um die Bahnkurve 10 abzufahren, muss der die axiale Verstellung eine Bahn beschreiben gemäß

$$\zeta(t) = \zeta_{max} \sin(\varpi \cdot t).$$

Für die Beschleunigung erhält man entsprechend

$$\ddot{\zeta}(t) = -\varpi^2 \cdot \zeta_{max} \sin(\varpi \cdot t)$$

Der für die Kurve K typische Radius R beträgt bei der Katarakt-Operation zwischen 2 und 3 mm. Damit beträgt der Umfang der Schnittfläche etwa 20 mm. Um eine gute Materialtrennung bei einer plasmablasenbasierten Technik zu erzielen, sollte der tangentiale Bahnabstand (benachbarte axiale Oszillationen) zwischen 1 und 10 μm betragen. Es sind also etwa zwischen 2.000 und 20.000 vertikale Schnittbahnen zu erzeugen. Innerhalb der Schnittbahnen sollten die Spotabstände in der Größenordnung zwischen 1 und 10 $\mu$m liegen.

Die Höhe H muss mindestens der Kapselsackdicke, also ca. 20 bis 25 $\mu$m entsprechen. Wenn sie geringer ist, können mehrere Schnittflächen 9 aufeinander "gestapelt" werden, um eine komplette Durchtrennung des Kapselsackes zu erreichen, wobei ein gewisser Überlapp sinnvoll sein kann.

Praktikabel erscheint eine Gesamthöhe von 25 $\mu$m bis 250 $\mu$m. Damit hat die Schnittfläche 9 einen Flächeninhalt von ca. 500.000 bis 5.000.000 $\mu$m$^2$. Mit einer Gitterkonstanten von 1x1 $\mu$m bis maximal 10x10 $\mu$m werden also 50.000 bis 5.000.000 Laserspots plaziert.

Bei einer Laserpulswiederholrate von 100 kHz ergibt sich eine minimale Schnittflächenerzeugungszeit (ohne Pausen, Totzeiten) von 0,5 bis 50 s. Da bei einer solchen Laserpulswiederholrate Pulsenergien im $\mu$J-Bereich leicht erzeugbar sind, ist es bevorzugt, einen größere mittlerem Spotabstand mit höherer Energie zu kombinieren, z. B. 0.5 $\mu$J und 3x3 $\mu$m. Damit beträgt die Schnittflächenerzeugungszeit auch bei großer Zylinderhöhe nur wenige Sekunden (weniger als 10 s).

Alternativ kann mit geringer Pulsenergie (<100 nJ) und einer Laserpulswiederholrate im Bereich einiger MHz ein Spotabstand von 1x1 $\mu$m verwendet werden. Beispielsweise ergibt sich bei 5 MHz ebenfalls wiederum eine Schnittflächenerzeugungszeit von nur wenigen Sekunden (weniger als 10 s).

Dies bedeutet, dass die axiale Verstellung innerhalb von ca. 5 Sekunden beim lateralen Umlauf um die Kurve K mit einem Umfang von 20 mm je nach Bahnabstand 2.000 bis 20.000 axiale Oszillationen realisieren muss. Die axiale Frequenz (Schwingungsfrequenz) beträgt damit 500 Hz bis 5 kHz. Für die Beschleunigung ergibt sich

$$Max(\ddot{\zeta}(t)) = \varpi^2 \cdot \zeta_{max} = 4\pi^2 f^2 \cdot \zeta_{max} \approx 10^{6...11} s^{-2} \cdot \zeta_{max}$$

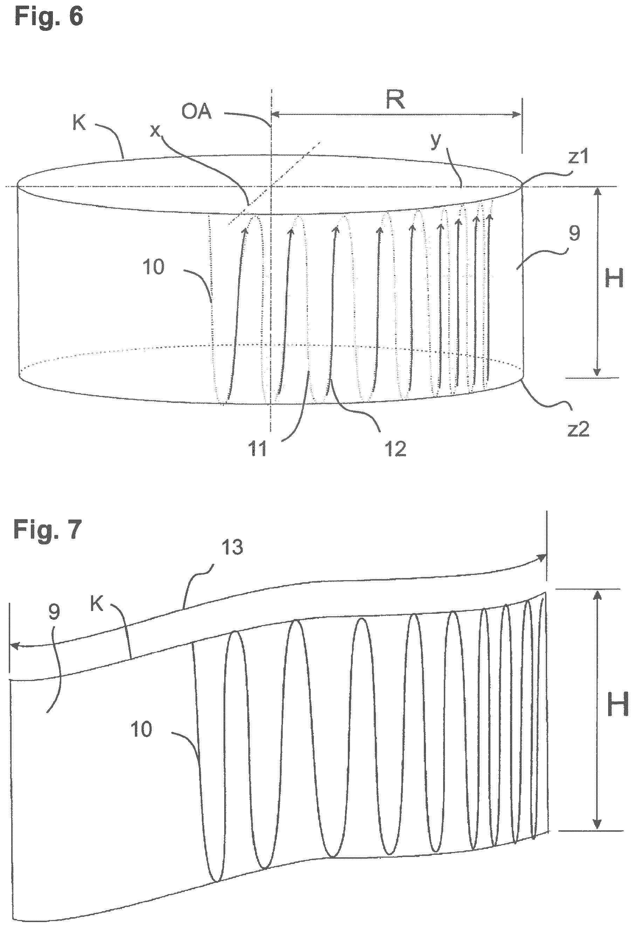

Zu beachten ist, dass die Art der Bahnkurve 10 eine ungünstige Beeinflussung des optischen Strahlweges durch das Material (Gewebe) durch vorangegangene Wechselwirkungsvorgänge nicht automatisch verhindert, wie dies bei einem schichtweisen Aufbau der Schnittfläche längs der Einfallsrichtung der Laserstrahlung der Fall wäre. Deshalb ist gemäß Fig. 6 eine Weiterbildung vorgesehen, indem die Laserstrahlung 2 auf Abschnitten 11 der Bahnkurve 10 in die Tiefe des Materials (weg von der Einfallsrichtung) so modifiziert wird, dass keine die Transmission nachfolgender Pulse nachteilig beeinflussende Wechselwirkung erfolgt, oder diese zumindest verringert wird. Im einfachsten Fall werden die Pulse auf diesen Abschnitten 11 vollständig unterdrückt. Dieses Konzept kann auch noch für die Bereiche in der Nähe der Umkehrpunkte der Bahn ausgedehnt werden, also bis zu etwa 40 % in der Umgebung der Wendepunkte der Bahnkurve 10 (nicht notwendigerweise symmetrisch).

Fig. 7 zeigt exemplarisch eine Schnittfläche 9, die im mathematischen Sinne einfach zusammenhängend ist, deren Kurve K also nicht zu einer Schleife geschlossen ist. Die Maximalausdehnung der Schnittfläche R ergibt sich damit durch die Länge 13 der Kurve K. Wiederum wird der Fokus lateral entlang der Kurve K verstellt. Während das Abfahrens der Länge 13 wird der Fokus zugleich axial oszillierend verstellt.

Die absolute Lage der axialen oberen und unteren Fokusposition variiert dabei längs der Kurve K. Dies ist natürlich nicht zwingend, es können auch konstante axiale obere und untere Fokuspositionen verwendet werden. Genauso wenig muss der Abstand zwischen der axialen oberen und unteren Fokusposition, zwischen denen die Oszillation ausgeführt wird, konstant sein. Im Ergebnis folgt die Lage des Fokus der in der Schnittfläche 9 mäandernden Bahnkurve 10, und die axiale obere(n) bzw. untere(n) Fokusposition(en) der Oszillation gibt (geben) den oberen bzw. unteren Rand der Schnittfläche 9 vor. Dies ist gleichermaßen für zweifach zusammenhängende Schnittflächen möglich.

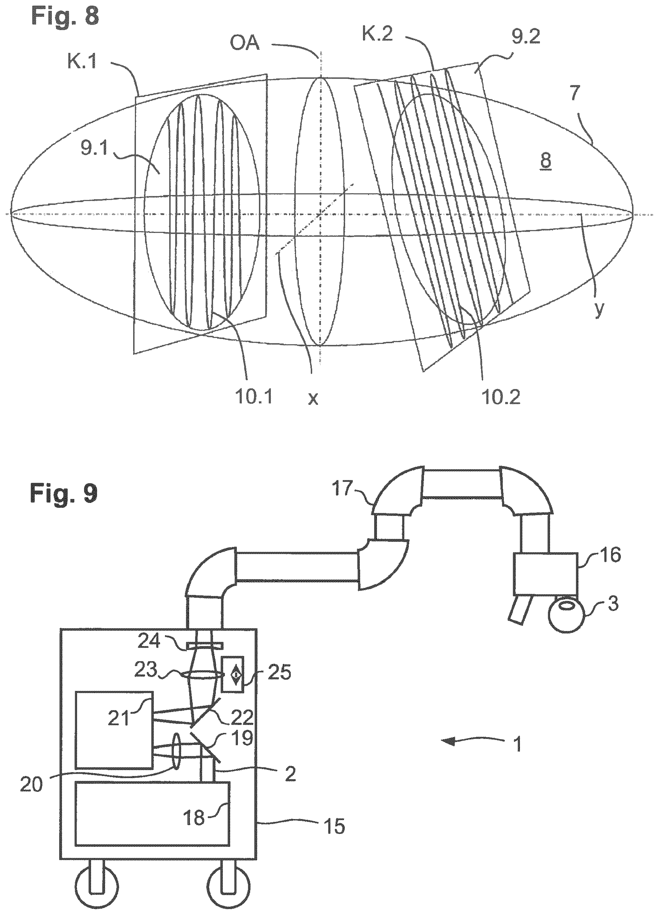

Fig. 8 zeigt exemplarisch mögliche Anwendungen für solche Schnittflächen. Es sind je zwei Schnittflächen wiederum an einem Kapselsack, umhüllend eine Augenlinse, dargestellt. Exemplarisch sind zwei Schnittflächen 9.1 und 9.2 gezeigt, die Kurven K.1 und K.2 haben.

Die linke Schnittfläche 9.1 ist ein Beispiel dafür, dass die oberen und untere Fokusposition während des Verschiebens entlang der Kurve K.1 variieren kann. Die Oszillation entlang der Bahnkurve 10.1 ist somit zur Lage längs der Kurve K.1 synchronisiert, so dass z. B. die in Fig. 8 gezeigte kreisscheibenförmige Schnittfläche erzeugt wird. Damit ist es, wie im Ausführungsbeispiel der Fig. 8 gezeigt, möglich, die Schnittfläche auf einen gewünschten Abschnitt des transparenten Materials zu beschränken, hier auf die Augenlinse, ohne andere Materialstrukturen zu beeinträchtigen, hier den Kapselsack.

Die rechte Schnittfläche 9.2 verdeutlicht, dass von einer strikten Parallelität der Schnittfläche zur optischen Achse OA auch abgewichen werden kann. Wie in der linken Schnittfläche 9.1 wird auch hier die axiale Fokusverstellung mit der lateralen Fokusverstellung synchronisiert. Nun jedoch dahingehend, dass eine zusätzlich laterale Fokusverstellung synchronisiert zur axialen Fokusverstellung ausgeführt wird, um die Schnittfläche 9.2 leicht gegen die optische Achse OA zu neigen. Natürlich kann diese Neigung auch auf Abschnitte der Schnittfläche 9.2 beschränkt sein. Besonders einfach ist diese zusätzliche laterale Fokusverstellung beim Behandlungsgerät der Fig. 2 dann zu erreichen, wenn die zusätzlich Fokusverstellung im Bildfeld B geeignet aktiviert und betätigt wird. Die Schnittfläche 9.2 ist immer noch im Wesentlichen parallel zur optischen Achse OA, da die zusätzliche laterale Fokusverstellung in Synchronisation zur axialen Fokusverstellung klein gegen die Ausdehnung der Kurve K ist.

Die axiale Fokusverschiebung kann einfach ausgeführt werden, wenn eine Minimierung der Amplitude erfolgen kann. Bevorzugt kann dies erfolgen, indem ein z-Scanner so gestaltet wird, dass das optische Übersetzungsverhältnis von Fokusbewegung zu z-Scanner-Bewegung weniger als 1:2 beträgt, vorzugsweise sogar weniger als 1:1. Das heißt, dass die mechanische Wegvariation im Scanner nicht größer ist als die Fokusbewegung im Objekt. Dann liegt dann die Beschleunigung im Bereich 0,1 bis $10^3$ m/s$^2$.

Ein optionales Mittel zur Erreichung eines solchen Wertes besteht darin, einen reflektiven z-Scanner zu verwenden, dessen optisches Design so beschaffen ist, dass vermieden ist, dass ein Strahlfokus bei einer üblichen Scan-Bahn auf eine optische Grenzfläche des Scanners positioniert wird.

Ein weiteres optionales Mittel besteht darin, dass der z-Scanner als Antrieb einen Piezo-Stack oder eine Tauchspule aufweist, die möglichst resonant betrieben werden. Auch eine Option ist es, die Reflektion mittels eines elektrooptischen Bauelements (z. B. AOM) zu bewirken.

Wie erwähnt, kann auch ein zweiter z-Scanner verwendet werden, der zusätzliche zeitlich langsame Divergenzänderungen (positiv oder negativ) des Laserstrahls realisiert, wobei die Ansteuerung der Scanner durch eine Steuereinheit erfolgt, die die Positionssignale (Messsignal) oder Steuersignale eines Scanners bei der Ansteuerung des jeweils anderen berücksichtigt, weil beispielsweise beide Steuersignale dort erzeugt werden. Die Steuereinheit kann durch die Steuereinrichtung 6 realisiert werden.

Für eine schnelle Variation der Fokuslage entlang der optischen Achse, d.h. für einen schnellen z-Scanner, im Bereich der ophthalmologischen Chirurgie, insbesondere für Katarakt-Operationen ist das in Fig. 9 schematisch dargestellte Behandlungsgerät 1 vorgesehen. In diesem Behandlungsgerät 1 sind Elemente, die bereits anhand der vorherigen Figuren erläutert wurde und die im Behandlungsgerät 1 funktions- oder strukturgleich sind, mit denselben Bezugszeichen versehen und werden deshalb nicht zwingend nochmals erläutert.

Das Behandlungsgerät 1 weist ein Basisteil 15 auf, welches die Laserstrahlung 2 bereitstellt und ein Handteil 16, an das die Laserstrahlung übertragen wird. Die Übertragung erfolgt mittels eines Gelenkarmes 17, der bevorzugt eine Freistrahloptik aufweist, die beispielsweise durch geeignete Umlenkspiegel (nicht dargestellt) realisiert sein kann. Die Verwendung von Lichtleitfasern ist nachteilig, da Standprobleme bezüglich der erforderlichen Strahlungsintensitäten der Laserstrahlung 2 bestehen. Lichtleitfasern sind im oder anstelle des Gelenkarms 17 dennoch möglich.

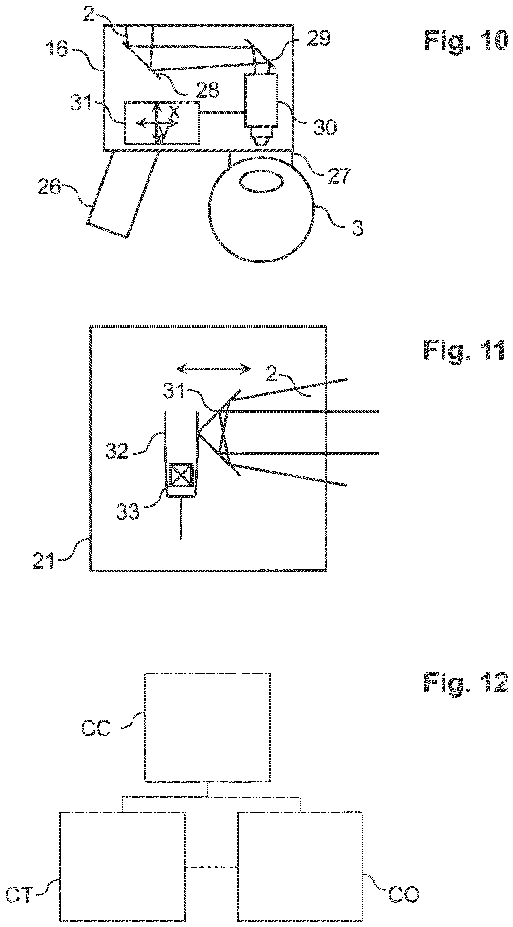

Das Handteil 16 wird auf das Auge 3 aufgesetzt. Es ist in Fig. 10 detailliert gezeigt und wird später noch erläutert werden.

Die z-Scanner und xy-Scanner sind im Basisteil 15 und im Handteil 16 realisiert. Beide Elemente bilden somit zusammen mit dem Gelenkarm 17 die Lasereinrichtung L. In der Bauweise der Fig. 9 ist der z-Scanner im Basisteil 15 vorgesehen. Eine Laserquelle 18 gibt die Laserstrahlung 2 ab, die über einen Umlenkspiegel 19 und eine Linse 20 in einem nicht-kollimierten Strahlengang in den z-Scanner 21 geleitet wird, dessen Aufbau später anhand der Figuren 11, 13, 14 noch erläutert werden wird. Aus dem z-Scanner gelangt die Laserstrahlung 2 über einen Umlenkspiegel 22 und Linsen 23, 24 als nicht-kollimiertes Bündel in den Gelenkarm 17. Die Linsen 23, 24 bilden ein Teleskop, das über einen Antrieb 25 verstellbar ist. Die Bedeutung dieses Teleskops wird noch erläutert werden.

Der Gelenkarm 17 beinhaltet bei Ausführung in Freistrahloptik eine Reihe von Umlenkspiegeln und gegebenenfalls eine Relayoptik, um das nicht-kollimierte Bündel zum Ausgang des Gelenkarms zu übertragen.

Das Handteil 16 empfängt die Laserstrahlung 2 als nicht-kollimiertes Bündel aus dem Gelenkarm 17 und gibt sie über ein Kontaktglas 27 in das Auge 3 ab. Zur Positionierung des Handteils 16 ist ein Griff 26 vorgesehen.

Die Laserstrahlung 2 wird im Handteil 16 über Umlenkspiegel 28 und 29 zu einem Objektiv 30 gebracht, das von einem Stellglied 31 quer zur optischen Achse verschoben wird. Dies realisiert die bereits erwähnte Bildfeldverstellung. Das Objektiv 30 ist kein Feldobjektiv, d. h. kein bildseitig telezentrisches Objektiv, vielmehr wird je nach Divergenz bzw. Konvergenz des Bündels an der Eingangspupille des Objektivs 30 die Laserstrahlung in unterschiedliche Tiefen im Auge 3 fokussiert. Damit wird die vom z-Scanner bewirkte Variation der Bündelausbreitung in eine Variation der axialen Fokusposition im Auge 3 umgesetzt.

Zur Verstellung des Bildfeldes wird das Objektiv 3 im Handteil 16 mit Hilfe des Stellgliedes 31 lateral bewegt. Zugleich sind die Umlenkspiegel 28 und 29 im Handteil 16 mechanisch angesteuert und werden von der Steuereinrichtung C der Verschiebung des Objektivs 30 so nachgeführt, dass die Laserstrahlung 2 als Bündel immer in der Eintrittspupille des Objektivs zentriert bleibt.

Fig. 11 zeigt schematisch die Wirkung des z-Scanners 21. Er verfügt über einen Eckspiegel 31, der einen Retro-Reflektor realisiert. Grundsätzlich könnte der Eckspiegel 31 durch einen andersartigen Retro-Reflektor ersetzt werden. Der Eckspiegel 31 ist an einer Stimmgabel 32 befestigt, welche von einem Erreger 33 in Schwingungen versetzt wird. Der Eckspiegel ist mit dem nicht-kollimierten Bündel der Laserstrahlung 2 beleuchtet. Durch die Verstellung der Position des Eckspiegels wird die Länge des nicht-kollimierten Strahlengangs verändert und damit die Ausbreitung des divergierenden Bündels der Laserstrahlung 2.

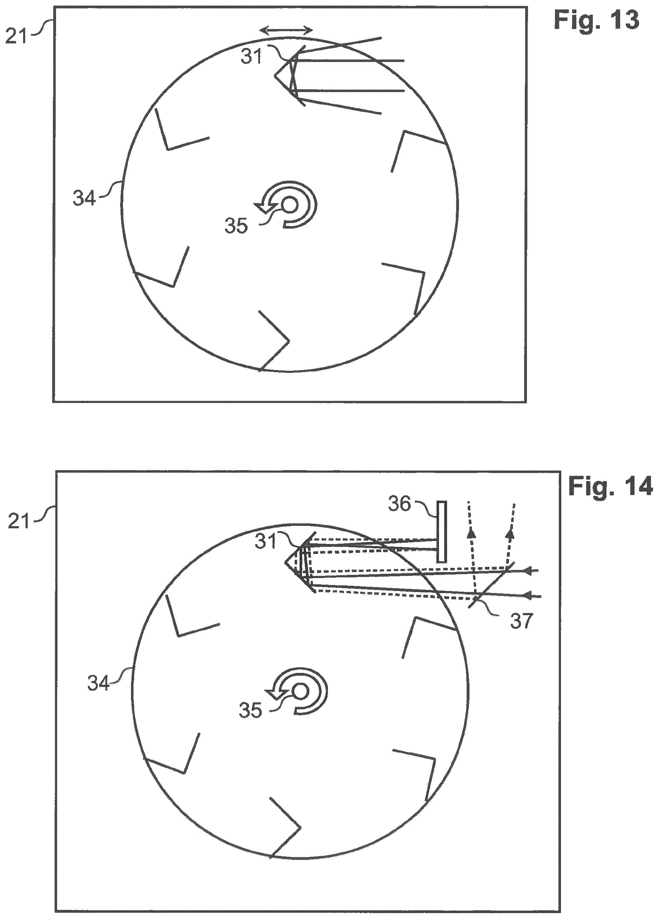

Die Fig. 13 und 14 zeigen Alternativen für den Scanner 21. Gemäß Fig. 13 sind eine Vielzahl von Eckspiegeln 31 auf einer rotierenden Scheibe 34 befestigt, die um eine Achse 35 in Rotation versetzt wird. Die Rotation bewirkt, dass die optische Weglänge des divergierenden Strahlenteils verstellt wird. Der Vorteil ist eine schnellere Verstellung, der Nachteil eine geringere Wiederholgenauigkeit und ein erhöhter Justieraufwand für die mehreren Eckspiegel 31. Fig. 14 zeigt eine nochmalige Weiterbildung, die zusätzlich zur Bauweise der Fig. 13 noch einen Endspiegel 36 sowie einen Strahlteiler 35 hat. Das einfallende divergierende Bündel der Laserstrahlung 2 durchläuft den Strahlteiler 37, wird am Eckspiegel 31 zum Endspiegel 36 umgelenkt, dort reflektiert (ab nun ist der Strahlengang gestrichelt gezeichnet), wiederum vom Eckspiegel 31 reflektiert und dann vom Strahlteiler 37 ausgekoppelt. Die Weglängenverstellung, die sich bei der Drehung der Scheibe 34 ergibt ist damit doppelt so groß, so dass bei gleicher Drehgeschwindigkeit der Scheibe 34 eine höhere Verstellgeschwindigkeit erreicht wird.

Um gezielte Schnitte im Auge gemäß der vorbeschriebenen Schnittführung durchzuführen, wird das Objektiv 31 im Handteil 16 vom Stellglied 31 (unter Nachführung der Umlenkspiegel 28 und 29) lateral bewegt. Während dieser lateralen Bewegung wird das Teleskop 23, 24 durch den Antrieb 25 synchron nachgestellt. Dies ist notwendig, weil durch die laterale Bewegung des Objektivs 31 die optische Weglänge vom Scanner 21 zum Objektiv 30 variiert wird. Da das Bündel der Laserstrahlung 2 auf das Objektiv 30 nicht-kollimiert einfällt, würde die Weglängenänderung zusätzlich zu einer Änderung der z-Position des Fokus führen. Die gewünschte z-Scannereigenschaft wäre damit gestört. Deshalb wird die Divergenz/Konvergenz des Lichtbündels mit Hilfe des Teleskops 23, 24 synchron zur Bewegung des Objektivs 30 verstellt.

Eine dafür vorgesehene steuerungstechnische Realisierung zeigt als Blockschaltbild die Fig. 12. Hier ist die Steuereinrichtung C in Form von drei Funktionen gezeigt. Ein zentraler Steuerblock CC steuert zwei Sub-Blöcke an, einen Steuerblock CT für die Ansteuerung des Teleskops 23, 24 und einen Steuerblock CO für das Stellglied 31, d.h. zur Verschiebung des Objektivs 30. Die steuerungstechnische Abstimmung der Verstellung der Querlage des Objektivs 30 und des Teleskops 23, 24 erreicht, dass die Lage des Fokus in Tiefenrichtung ausschließlich durch den z-Scanner 21 eingestellt wird, und dass die Verschiebung des Bildfeldes B keine gleichzeitige Verschiebung der Tiefenlage des Fokus mit sich bringt. Mit anderen Worten, die synchrone Verstellung von Teleskop 23, 24 als Mittel zum Beeinflussen der Divergenz/Konvergenz der auf das Objektiv 30 einfallenden Strahlung und die Querverschiebung des Objektivs sorgen dafür, dass die Verschiebung des Bildfeldes in einer Ebene senkrecht zur optischen Achse und nicht auf einer gekrümmten Bahn erfolgt.

Bei der Katarakt-Behandlung ist es vorteilhaft, Navigationseigenschaften bereitzustellen, welche die Lage der zu bearbeitenden Strukturen, beispielsweise die Lage des Kapselsacks oder der Linse aufzufinden erlaubt. Bei einem System mit einer mechanisch bewegten Optik zur lateralen Fokusverschiebung, die ein Bildfeld hat, welches kleiner ist als die zu findende Struktur oder die zu erzeugende Schnittfläche, kommen verschiedene Varianten in Frage.

Variante 1 - Konfokale Detektion: Zur Vermessung der Topographie des Auges 3 im Bereich der Hornhaut 5 und/oder der Linse 8 wird rückreflektiertes Licht mit Hilfe von Polarisationsoptiken umgeleitet, auf einer Blende fokussiert und mit Hilfe eines Photodetektors erfasst. Für diese Messung wird die Wellenlänge der Laserstrahlung 2 verwendet. Mit Hilfe des z-Scanners werden konfokale Signale von einem kleinen Scanbereich 10-100 $\mu$m erfasst. Die vom Photodetektor erfassten Signale werden verstärkt, z. B. mit einem Lock-In-Verfahren oder Boxcar Integrator. Die Referenzfrequenz für den Lock-in-Verstärker stimmt mit der Scanfrequenz des schnellen z-Scanners überein. Die konfokalen Signale werden während der (langsamen) Bewegung des Objektivs erfasst. Das Bildfeld B bewegt sich entlang der Kurve K, welches mit dem Muster für die Laserbehandlung übereinstimmt. Gleichzeitig wird die axiale Fokuslage verstellt, so dass eine Bahnkurve (z. B. die Bahnkurve 10) abgefahren wird. In dieser Art und Weise wird die genaue Position ermittelt, wo eine Laserdesorption erfolgen soll, und damit wird die Sicherheit der Behandlung erhöht.

Variante 2 - OCT Detektion: Bei dieser Variante wird die Topographie des Auges 3 im Bereich der Hornhaut 5 und/oder der Linse 8 mit kurzkohärentem Licht vermessen, welches eine andere Wellenlänge aufweist. Das Licht einer kurzkohärenten Quelle wird mit Hilfe eines dichroitischen Spiegels in die Optik O eingekoppelt. Das von Augenstrukturen rückreflektierte Licht wird durch denselben dichroitischen Spiegel umgeleitet und mit Hilfe einer interferometrischen Anordnung detektiert. Um die Sicherheit und Präzision der Laserbehandlung zu erzielen, wird ein Objektiv eingesetzt, welches eine numerische Apertur im Bereich 0,15-0,2 aufweist. Bei einer vollausgeleuchteten Eintrittspupille des Objektivs ist der z-axiale Messbereichs der OCT-Detektion durch die Schärfetiefe des Objektivs eingeschränkt. Demgemäß wird hierbei die kurzkohärente Beleuchtung so ausgelegt (z. B. durch entsprechende Auswahl der Kollimatorgeometrie oder mit Hilfe ein Blende), dass nur ein Teil der Eintrittspupille des Objektiv O ausgeleuchtet wird. Da die effektive numerische Apertur des fokussierten kurzkohärenten Lichtes dadurch verringert wird, wird hierbei ein größerer Tiefenbereich für die OCT-Detektion realisiert.

Es erfolgt bei diesen Navigationsmessungen bevorzugt keine tomographische Bildaufnahme der Augenstrukturen, sondern anhand tiefenaufgelöster Messungen an ausgewählte Stellen wird die Augenstruktur simuliert und an ein rechnerisches Augenmodell angepasst. Das Ergebnis dieser Anpassung wird als optionale Animation dargestellt. Anhand dieser Animation wird der Chirurg in der Lage versetzt, die räumlichen Grenzen der Photodesorption einzustellen.

Vorzugsweise erfolgen die tiefenaufgelösten Navigationsmessungen entlang der Bahnkurve, welche für die Laserbehandlung eingesetzt wird. Wenn anhand dieser Messungen eine Dezentrierung der kreisförmigen Musters der geplanten Kapselsacköffnung in Bezug auf der Augenlinse festgestellt wird, kann ggf. eine neue Navigationsmessreihe erfolgen. Die hiermit gewonnenen Daten werden mit dem rechnerischen Augenmodell erneut korreliert und das Ergebnis wird als Animation zur Kontrolle der Laserbehandlung angezeigt.

**Patentansprüche**

1. Bearbeitungsvorrichtung zur Erzeugung einer Schnittfläche in einem transparenten Material (7, 8), die umfasst

   - eine Lasereinrichtung (L), die dazu ausgebildet ist, mittels optischer Strahlung (2) eine Trennung innerhalb des transparenten Materials (7, 8) zu bewirken, und die aufweist

     - eine Optik (O), die optische Strahlung längs einer optischen Achse (OA) in einen im Material gelegenen Fokus (6) bündelt und im Material (7, 8) ein Bildfeld hat (B), in dem der Fokus (6) liegt und das eine Bildfeldgröße hat,
     - eine Einrichtung zur Verstellung einer Lage des Fokus (6) quer zur optischen Achse (OA) und längs der optischen Achse (OA),

   - eine Steuereinrichtung (S), welche mit der Lasereinrichtung (L) verbunden ist und die Lasereinrichtung (L) so ansteuert, daß deren Einrichtung zur Verstellung der Lage des Fokus (6) die Lage des Fokus (6) der optischen Strahlung (2) im Material (7, 8) entlang einer Bahnkurve (10) verschiebt, wobei die Steuereinrichtung (S) die Lasereinrichtung (L) so ansteuert, daß die Schnittfläche (10) sich im Wesentlichen parallel zur optischen Achse (OA) erstreckt und in Projektion längs der optischen Achse (OA) eine Kurve (K) ist, die längs der optischen Achse (OA) eine Maximalausdehnung aufweist, und die Steuereinrichtung (S) längs der optischen Achse (OA) während der Bewegung auf der Kurve (K) eine mehrfach oszillierende Verstellung die Lage des Fokus (6) zwischen einer oberen axialen Fokusposition (z1) und einer unteren axialen Fokusposition (z2) vorgibt,

   **dadurch gekennzeichnet, dass**

     - die Einrichtung zur Verstellung der Lage des Fokus (6) quer zur optischen Achse (OA) eine Verschiebung des Bildfeldes (B) quer zur optischen Achse (OA) bewirkt,
   - die Steuereinrichtung (S) die Lasereinrichtung (L) so ansteuert, dass die Schnittfläche (9) quer zur optischen Achse (OA) eine Maximalausdehnung hat, die größer ist als die Bildfeldgröße, und für die Einrichtung zur Verstellung der Lage des Fokus (6) quer zur optischen Achse (OA) eine Bewegung der Lage des Fokus (6) entlang der Kurve (K) vorgibt und
   - die Lasereinrichtung (L) ein Basisteil (15), ein bewegliches Handteil (16) und eine flexible oder gelenkige, optische Übertragungseinrichtung (17) aufweist, welche das Basisteil (15) und das Handteil (16) verbindet, wobei das Basisteil (15) die Laserstrahlung (2) in die Übertragungseinrichtung (17) als nicht-kollimiertes Bündel einkoppelt, die Übertragungseinrichtung (17) das nicht-kollimierte Bündel zum Handteil (16) leitet, das Handteil (16) ein Objektiv (30) aufweist, welches das nicht-kollimierte Bündel aufnimmt und in das Auge (3) fokussiert, und die Einrichtung zur Verstellung der Lage des Fokus (6) zweiteilig ausgebildet ist und einen ersten Scanner (21), der die Lage des Fokus (6) längs der optischen Achse (OA) verstellt und im Basisteil (15) angeordnet ist, und einen zweiten Scanner (31, 23, 24, 25) aufweist, der die Lage des Fokus (6) quer zur optischen Achse (OA) verstellt, wobei der zweite Scanner ein Stellglied (31) zur Verschiebung des Objektivs (30) quer zur optischen Achse (OA) und eine Divergenzverstelleinrichtung (23-25) umfasst, welche eine Divergenz oder Konvergenz des nicht-kollimierten Bündels beeinflusst, und wobei weiter die Steuereinrichtung so ausgebildet ist, dass sie die die Divergenzverstelleinrichtung (23-25) synchron mit dem Stellglied (31) zur Verschiebung des Objektivs (30) ansteuert.

2. Vorrichtung nach Anspruch 1, **dadurch gekennzeichnet, dass** die Steuereinrichtung (S) auf Abschnitten (11) der Bahnkurve (10), in den sich die Lage des Fokus (6) mit einer Einfallsrichtung der optischen Strahlung (2) bewegt, die optische Strahlung (2) abschaltet oder so modifiziert, daß die optische Strahlung (2) keine Materialtrennungswirkung im transparenten Material (7, 8) hat.

3. Vorrichtung nach Anspruch 2, **dadurch gekennzeichnet, dass** die Oszillation asymmetrisch ist, wobei die Abschnitte (11) der Bahnkurve (10), in den sich die Lage des Fokus (6) mit der Einfallsrichtung der optischen Strahlung (2) bewegt, steiler verlaufen, als Abschnitte (12) der Bahnkurve (10), in den sich die Lage des Fokus (6) entgegen der Einfallsrichtung der optischen Strahlung (2) bewegt.

4. Vorrichtung nach Anspruch 1, **dadurch gekenn-**

**zeichnet, dass** die Divergenzverstelleinrichtung (23-25) im Basisteil (15) angeordnet ist und/oder ein Teleskop (23, 24) und einen dieses verstellenden Aktuator (25) aufweist.

5. Vorrichtung nach Anspruch 1 oder 4, **dadurch gekennzeichnet, dass** der erste Scanner (21) zur Verstellung der Lage des Fokus (6) längs der optischen Achse (OA) eine Weglänge des nicht-kollimierten Bündels verändert, bevorzugt mittels mindestens einen verschieblichen Eckspiegels (31).

6. Vorrichtung nach Anspruch 5, **dadurch gekennzeichnet, dass** der erste Scanner (21) einen Schwinger (32) zur periodischen Veränderung der Weglänge aufweist oder mehrere Eckspiegel (31) aufweist, die auf einer rotierenden Scheibe (34) montiert sind.

7. Vorrichtung nach einem der Ansprüche 4 bis 6, **dadurch gekennzeichnet, dass** das Handstück eine Nachführeinrichtung (28, 29) aufweist, die synchron zur Verschiebung des Objektivs (30) das nicht-kollimierte Bündel in einer Pupille des Objektivs (30) zentriert hält.

8. Verfahren oder Vorrichtung nach einem der obigen Ansprüche, **dadurch gekennzeichnet, dass** Schnittfläche (10) zylindermantelförmig ist und/oder dass die obere axiale Fokusposition (z1) einen oberen Rand der Schnittfläche (10) und die untere axiale Fokusposition (z2) einen unteren Rand der Schnittfläche (10) definiert.

9. Verfahren oder Vorrichtung nach einem der obigen Ansprüche, **dadurch gekennzeichnet, dass** die Verschiebung des Bildfeldes (B) durch eine Bewegung der Optik (O) quer zur optischen Achse (OA) bewirkt ist.

10. Verfahren oder Vorrichtung nach einem der obigen Ansprüche, **dadurch gekennzeichnet, dass** die Kurve (K) eine periodische Lissajous-Figur ist.

11. Verfahren oder Vorrichtung nach einem der obigen Ansprüche, **dadurch gekennzeichnet, dass** die Kurve (K) eine geschlossene Kurve ist.

## Claims

1. A treatment apparatus for producing a cut surface in a transparent material (7, 8), which apparatus comprises

    - a laser device (L) which is adapted to generate a separation within the transparent material (7, 8) by means of optical radiation (2) and which laser device comprises

    - optics (O) which focus the optical radiation along an optical axis (OA) into a focus (6) situated in the material and which have in the material (7, 8) an image field (B) in which the focus (6) lies and which have an image field size, and
    - a focus adjustment device for adjusting a position of the focus (6) transverse to the optical axis (OA) and along the optical axis (OA),

    - a control device (S) which is connected to the laser device (L) and controls the laser device (L) such that the focus adjustment device moves the position of the focus (6) of the optical radiation (2) in the material (7, 8) along a path (10), wherein the control device (S) controls the laser device (L) such that the cut surface (10) extends substantially parallel to the optical axis (OA) and, in projection along the optical axis (OA), is a curve (K) which has a maximum extent (R) along the optical axis (OA), wherein, during the movement along the curve (K), the control device (S) defines the position of the focus (6) by a multiple oscillating adjustment of the position of the focus (6) along the optical axis (OA) between an upper axial focus position (z1) and a lower axial focus position (z2),

**characterized in that**

    - the focus adjustment device moves the position of the focus (6) transverse to the optical axis (OA) by a shift of the image field (B) transverse to the optical axis (OA),
    - the control device (S) controls the laser device (L) such that the cut surface (9), transverse to the optical axis (OA), has a maximum extent which is greater than the image field size and that the focus adjustment device defines a movement of the position of the focus (6) transverse to the optical axis (OA) along the curve (K), and
    - the laser device (L) has a base part (15), a movable handpiece (16) and a flexible or articulated, optical transmission device (17), which connects the base part (15) to the handpiece (16), wherein the base part (15) couples the laser radiation (2) into the transmission device (17) as a non-collimated beam, the transmission device (17) transmits the non-collimated beam to the handpiece (16), the handpiece (16) comprises an objective lens (30), which receives the non-collimated beam and focuses it into the eye (3), and the focus adjustment device for moving the position of the focus (6) is formed by two parts and comprises a first scanner (21), which moves the position of the focus (6) along the optical axis (OA) and is arranged in the base part (15), and a second scanner (31, 23, 24, 25), which moves the position of the focus (6) transverse to the optical axis (OA), wherein the second scanner comprises an actuator (31) for displacing the objective lens (30) transverse to the optical axis (OA) and a divergence-adjusting device (23-25), which changes a divergence or convergence of the non-collimated beam, and wherein furthermore the control device is designed such that it controls the divergence-adjusting device (23-25) to act synchronously with the actuator (31) for displacing the objective lens (30).

2. The apparatus according to claim 1, **characterized in that**, while the focus is on sections (11) of the path (10) in which the position of the focus (6) moves with a direction of incidence of the optical radiation (2), the control device (S) switches off or modifies the optical radiation (2) such that the optical radiation (2) has no material-cutting effect in the transparent material (7, 8).

3. The apparatus according to claim 2, **characterized in that** the oscillation is asymmetrical, wherein the sections (11) of the path (10) in which the position of the focus (6) moves with the direction of incidence of the optical radiation (2) run more steeply than other sections (12) of the path (10) in which the position of the focus (6) moves contrary to the direction of incidence of the optical radiation (2).

4. The apparatus according to claim 1, **characterized in that** the divergence-adjusting device (23-25) is arranged in the base part (15) and/or comprises a telescope (23, 24) and an actuator (25) that adjusts it.

5. The apparatus according to claim 1 or 4, **characterized in that** for moving the position of the focus (6) along the optical axis (OA) the first scanner (21) changes a path length of the non-collimated beam, preferably by means of at least one displaceable corner mirror (31).

6. The apparatus according to claim 5, **characterized in that** the first scanner (21) comprises an oscillator (32) for periodically changing the path length or the first scanner (21) comprises several corner mirrors (31), which are mounted on a rotating disc (34).

7. The apparatus according to any of claims 4 to 6, **characterized in that** the handpiece comprises a repositioning device (28, 29) which holds the non-collimated beam centred in a pupil of the objective lens (30) synchronously with the displacement of the objective lens (30).

8. The apparatus according to any of the above claims, **characterized in that** cut surface (10) has a shape of a cylinder jacket and/or the upper axial focus position (z1) defines an upper edge of the cut surface (10) and the lower axial focus position (z2) defines a lower edge of the cut surface (10).

9. The apparatus according to any of the above claims, **characterized in that** the optics (O) is moved transverse to the optical axis (OA) to shift the image field (B).

10. The apparatus according to any of the previous claims, **characterized in that** the curve (K) is a periodic Lissajous figure.

11. The apparatus according to any of the previous claims, **characterized in that** the curve (K) is a closed curve.

**Revendications**

1. Dispositif de traitement destiné à produire une surface de coupe dans un matériau transparent (7, 8) qui comprend

    - un dispositif laser (L) conçu pour provoquer une séparation à l'intérieur du matériau transparent (7, 8) au moyen d'un rayonnement optique (2), et comportant
    - une optique (O) qui focalise le rayonnement optique le long d'un axe optique (OA) dans un foyer (6) situé dans le matériau et qui présente dans le matériau (7, 8) un champ image (B) dans lequel est situé le foyer (6) et ayant une taille de champ image,
    - un dispositif destiné à ajuster une position du foyer (6) transversalement à l'axe optique (OA) et le long de l'axe optique (OA),
    - un dispositif de commande (S) qui est relié au dispositif laser (L) et commande le dispositif laser (L) de manière à ce que son dispositif de réglage de la position du foyer (6) déplace la position du foyer (6) du rayonnement optique (2) dans le matériau (7, 8) le long d'une trajectoire (10), dans lequel le dispositif de commande (S) commande le dispositif laser (L) de manière à ce que la surface de coupe (10) s'étende sensiblement parallèlement à l'axe optique (OA) et à ce qu'elle soit, en projection le long de l'axe optique (OA), une courbe (K) ayant une étendue maximale le long de l'axe optique (OA) et le dispositif de commande (S) faisant en sorte que, pendant le mouvement sur la courbe (K) le long de l'axe optique (OA), un réglage oscillatoire multiple prédétermine la position du foyer (6) entre une position focale axiale supérieure (z1) et une position focale axiale inférieure (z2),

**caractérisé en ce que**

- le dispositif de réglage de la position du foyer (6) transversalement à l'axe optique (OA) provoque un déplacement du champ image (B) transversalement à l'axe optique (OA),
- le dispositif de commande (S) commande le dispositif laser (L) de manière à ce que la surface de coupe (9) présente transversalement à l'axe optique (OA) une étendue maximale supérieure à la taille du champ image et prédétermine, pour le dispositif de réglage de la position du foyer (6) transversalement à l'axe optique (OA), un mouvement de la position du foyer (6) le long de la courbe (K), et
- le dispositif laser (L) comprend une pièce de base (15), une pièce à main (16) mobile et un dispositif de transmission optique (17) flexible ou articulé, lequel relie la pièce de base (15) et la pièce à main (16), dans lequel la pièce de base (15) injecte le rayonnement laser (2) dans le dispositif de transmission (17) sous la forme d'un faisceau non collimaté, le dispositif de transmission (17) guide le faisceau non collimaté vers la pièce à main (16), la pièce à main (16) comporte un objectif (30) qui reçoit le faisceau non collimaté et le focalise dans l'œil (3), et le dispositif d'ajustement de la position du foyer (6) est réalisé en deux parties et comporte un premier dispositif de balayage (21) qui ajuste la position du foyer (6) le long de l'axe optique (OA) et qui est disposé dans la pièce de base (15), et un second dispositif de balayage (31, 23, 24, 25) qui ajuste la position du foyer (6) transversalement à l'axe optique (OA), dans lequel le second dispositif de balayage comprend un organe de réglage (31) destiné à déplacer l'objectif (30) transversalement à l'axe optique (OA) et un dispositif de réglage de divergence (23-25) qui agit sur une divergence ou une convergence du faisceau non collimaté, et dans lequel le dispositif de commande est en outre réalisé de manière à commander le dispositif d'ajustement de divergence (23-25) en synchronisme avec l'actionneur (31) pour déplacer l'objectif (30).

2. Dispositif selon la revendication 1, **caractérisé en ce que** le dispositif de commande (S) se déplace sur des sections (11) de la trajectoire (10) dans lesquelles la position du foyer (6) se déplace avec une direction d'incidence du rayonnement optique (2) qui occulte le rayonnement optique (2) ou le modifie de manière à ce que le rayonnement optique (2) n'ait pas d'effet de séparation des matériaux dans le matériau transparent (7, 8).

3. Dispositif selon la revendication 2, **caractérisé en ce que** l'oscillation est asymétrique, les sections (11) de la trajectoire (10) dans lesquelles la position du foyer (6) se déplace avec la direction d'incidence du rayonnement optique (2) présentant une inclinaison plus prononcée que les sections (12) de la trajectoire (10) dans lesquelles la position du foyer (6) se déplace en sens opposé à la direction d'incidence du rayonnement optique (2).

4. Dispositif selon la revendication 1, **caractérisé en ce que** le dispositif de réglage de divergence (23-25) est disposé dans la pièce de base (15) et/ou comporte un télescope (23, 24) et un actionneur (25) réglant ce dernier.

5. Dispositif selon la revendication 1 ou 4, **caractérisé en ce que** le premier dispositif de balayage (21) modifie une longueur de trajet du faisceau non collimaté, de préférence au moyen d'au moins un miroir de renvoi (31) coulissant, pour ajuster la position du foyer (6) le long de l'axe optique (OA).

6. Dispositif selon la revendication 5, **caractérisé en ce que** le premier dispositif de balayage (21) comporte un dispositif oscillant (32) destiné à faire varier périodiquement la longueur de trajet ou plusieurs miroirs de renvoi (31), lesquels sont montés sur un disque rotatif (34).

7. Dispositif selon l'une des revendications 4 à 6, **caractérisé en ce que** la pièce à main comprend un dispositif de poursuite (28, 29) qui, en synchronisme avec le déplacement de l'objectif (30), maintient le faisceau non collimaté centré dans une pupille de l'objectif (30).

8. Dispositif selon l'une des revendications précédentes, **caractérisé en ce que** la surface de coupe (10) présente la forme d'une gaine cylindrique et/ou **en ce que** la position focale axiale supérieure (z1) définit un bord supérieur de la surface de coupe (10) et la position focale axiale inférieure (z2) définit un bord inférieur de la surface de coupe (10).

9. Dispositif selon l'une des revendications précédentes, **caractérisé en ce que** le déplacement du champ image (B) est effectué par un mouvement transversal de l'optique (O) par rapport à l'axe optique (OA).

10. Dispositif selon l'une des revendications précédentes, **caractérisé en ce que** la courbe (K) est une figure de Lissajous périodique.

11. Dispositif selon l'une des revendications précédentes, **caractérisé en ce que** la courbe (K) est une courbe fermée.

EP 2 768 447 B2

# Fig. 1

# Fig. 3

# Fig. 2

## Fig. 4

## Fig. 5

**Fig. 6**

**Fig. 7**

**Fig. 8**

**Fig. 9**

Fig. 10

Fig. 11

Fig. 12

Fig. 13

Fig. 14

**EP 2 768 447 B2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110022036 A1
- EP 1486185 A
- US 7486409 B
- US 6590670 B
- DE 10034251
- EP 1159986 A2
- US 5549632 A
- DE 69500997 T2
- WO 2004032810 A2